Europäisches Patentamt

European Patent Office

Office européen des brevets

(11) Numéro de publication : **0 478 472 B1**

(12) **FASCICULE DE BREVET EUROPEEN**

(45) Date de publication du fascicule du brevet :
03.08.94 Bulletin 94/31

(51) Int. Cl.$^5$ : **C07C 55/14,** C07C 51/14,
C07C 67/38, C07C 69/44

(21) Numéro de dépôt : **91420326.0**

(22) Date de dépôt : **13.09.91**

(54) **Procédé de préparation de l'acide adipique par hydrocarboxylation d'acides penténiques.**

(30) Priorité : **28.09.90 FR 9012214**

(43) Date de publication de la demande :
01.04.92 Bulletin 92/14

(45) Mention de la délivrance du brevet :
03.08.94 Bulletin 94/31

(84) Etats contractants désignés :
BE DE ES FR GB IT NL

(56) Documents cités :
EP-A- 0 188 209
EP-A- 0 274 076

(73) Titulaire : **RHONE-POULENC CHIMIE**
**25, quai Paul Doumer**
**F-92408 Courbevoie Cédex (FR)**

(72) Inventeur : **Denis, Philippe**
**16, Allées des Troenes**
**F-69150 Decines (FR)**
Inventeur : **Grosselin, Jean-Michel**
**12, Allée des Erables**
**F-69340 Francheville (FR)**

(74) Mandataire : **Vignally, Noel et al**
**Rhône-Poulenc Chimie**
**Direction de la Propriété Industrielle**
**Centre de Recherches des Carrières**
**B.P. 62**
**F-69192 Saint-Fons Cédex (FR)**

## Description

La présente invention concerne un procédé de préparation de l'acide adipique par hydrocarboxylation d'acides pénténiques, c'est-à-dire par réaction de l'eau et de l'oxyde de carbone sur au moins un acide pénténique, en présence d'un catalyseur à base de rhodium et d'au moins un promoteur iodé.

Dans la demande de brevet européen n° 188 209 il est proposé un procédé de préparation d'acides dicarboxyliques linéaires, en particulier de l'acide adipique par réaction d'acides monocarboxyliques insaturés, en particulier l'acide pentène-3 oïque, de l'oxyde de carbone et d'eau en présence d'un catalyseur à base de rhodium et d'un promoteur iodé, la réaction étant conduite dans un solvant tel le chlorure de méthylène à une température de 100 à 240°C et sous une pression totale comprise entre 14 et 240 atm ; une température comprise entre 150 et 180°C et une pression totale comprise entre 24 et 40 atmosphères sont estimées préférables. La pression partielle du monoxyde de carbone est habituellement comprise entre 10 et 35 atm et de préférence entre 10 et 17 atm. Le choix du solvant est indiqué être critique dans le cadre du procédé en cause.

Il est affirmé que des solvants non polaire tels le cyclohexane et le toluène sont indésirables en raison de leur propension à promouvoir directement la formation de produits branchés et indirectement celle des acides monocarboxyliques saturés.

Dans la demande de brevet européen n° 0 274 076, il est proposé un procédé de préparation d'acides carboxyliques linéaires par hydroxycarboxylation d'esters insaturés ou d'alcènes à insaturation terminale comportant de 4 à 16 atomes de carbone en présence d'un catalyseur à base de rhodium et d'un promoteur iodé. La réaction est conduite dans un solvant choisi indifféremment parmi le chlorure de méthylène, le dichloro-1,2 éthane et les solvants aromatiques et, un acide aliphatique ou aromatique de pKa compris entre 4,2 et 5,2 est présent comme accélérateur de la réaction. La pression partielle de l'oxyde de carbone est comprise entre 10 et 200 et, de préférence entre 13 et 20 atm.

Toutefois au départ d'esters pénténiques on observe essentiellement la formation de monoadipate de méthyle.

Il a maintenant été trouvé qu'il est possible d'obtenir sélectivement l'acide adipique par hydrocarboxylation d'acides pénténiques en présence d'un catalyseur à base de rhodium et d'un promoteur iodé dans un solvant choisi dans le groupe constitué par les hydrocarbures aliphatiques ou cycloaliphatiques, saturés, les hydrocarures aromatiques et leurs dérivés chlorés.

La présente invention a donc pour objet un procédé de préparation de l'acide adipique par réaction de l'eau et de l'oxyde de carbone sur au moins un acide pénténique, en présence d'un catalyseur à base de rhodium et d'au moins un promoteur iodé, à une température comprise entre 100 et 240°C, sous une pression supérieure à la pression atmosphérique caractérisé en ce que

a) la réaction est mise en oeuvre dans au moins un solvant choisi dans le groupe constitué par les hydrocarbures aliphatiques ou cycloaliphatiques, saturés, les hydrocarbures aromatiques et les hydrocarbures cycloaliphatiques ou aromatiques chlorés et en ce que

b) la pression partielle de l'oxyde de carbone mesurée à 25°C est inférieure à 12 bar.

La présente invention concerne également un procédé tel que décrit précédemment, mais caractérisé en ce que la réaction est mise en oeuvre dans un hydrocarbure aliphatique chloré et en ce que la pression partielle de l'oxyde de carbone mesurée à 25°C est inférieure ou égale à 8 bar.

Par acide pénténique, on entend dans le cadre de la présente invention l'acide pentène-2 oïque, l'acide pentène-3 oïque, l'acide pentène-4 oïque et leurs mélanges.

L'acide pentène-4 oïque conduit à de bons résultats, mais reste peu disponible.

L'acide pentène-3 oïque pris isolément ou en mélange avec ses isomères est plus particulièrement approprié compte-tenu de son accessibilité et des résultats satisfaisants auxquels il conduit dans le cadre du présent procédé.

Le procédé selon la présente invention nécessite la présence d'un catalyseur à base de rhodium. N'importe quelle source de rhodium est susceptible d'être mise en oeuvre.

A titre d'exemples de sources de rhodium susceptibles de convenir à la mise en oeuvre du présent procédé on peut citer :

Rh métal ; $Rh_2O_3$ ;

$RhCl_3$3 ; $RhCl_3$, $3H_2O$ ;

$RhBr_3$ ; $RhBr_3$, $3H_2O$ ;

$RhI_3$ ; $Rh(NO_3)_3$ ; $Rh(NO_3)_3$, $2H_2O$ ;

$Rh_2(CO)_4Cl_2$ ; $Rh_2(CO)_4Br_2$ ; $Rh_2(CO)_4I_2$ ;

$Rh(CO)Cl[P(C_6H_5)_3]_2$

$Rh[P(C_6H_5)_3]_2(CO)I$

$Rh[P(C_6H_5)_3]_3Br$

$Rh_4(CO)_{12}$ ; $Rh_6(CO)_{16}$ ; $Rh(CO)_2$ (acac) ;

$Rh(Cod)(acac)_2$ ; $Rh(acac)_3$ ;

$Rh_2(Cod)_2Cl_2$ . $Rh_2(CO_2CH_3)_4$ ;

$HRh(CO)[P(C_6H_5)_3]_3$ ;

(Cod = cyclooctadiène-1,5 ; acac : acétylacétonate)

Conviennent plus particulièrement à la mise en oeuvre du présent procédé :

$HRh(CO)[P(C_6H_5)_3]_3$ ;

$Rh(CO)Cl[P(C_6H_5)_3]_2$ ;

$Rh_2(Cod)_2Cl_2$ ;

$Rh_2(CO)_4Cl_2$ ;

$RhI_3$ ; $RhCl_3, 3H_2O$ ; $Rh(acac)_3$ ;

$Rh(Cod)(acac)_2$ ; $Rh_2(CO_2CH_3)_4$ ; $Rh_4(CO)_{12}$ ;

et $Rh_6(CO)16$.

La quantité de rhodium à mettre en oeuvre peut varier dans de larges limites.

En général, une quantité exprimée en mole de rhodium métallique par litre de milieu réactionnel comprise entre $10^{-3}$ et $10^{-1}$ conduit à des résultats satisfaisants. Des quantités inférieures peuvent être mise en oeuvre ; on observe toutefois que la vitesse de réaction est faible. Des quantités supérieures n'ont d'inconvénients qu'au plan économique.

De préférence, la concentration de rhodium est comprise entre $5.10^{-3}$ et $10^{-2}$ (inclus) mol/l.

Par promoteur iodé on entend dans le cadre du présent procédé HI et les composés organoiodés capables de générer HI dans les conditions réactionnelles et, en particulier les iodures d'alkyles en $C_1$-$C_{10}$, l'iodure de méthyle étant plus particulièrement préconisé.

La quantité de promoteur iodé à mettre en oeuvre est en général telle que le rapport molaire I/Rh soit supérieur ou égal à 0,1. Il n'est pas souhaitable que ce rapport excède 20. De préférence, le rapport molaire I/Rh est compris entre 1 et 4 (inclus).

La présence d'eau est indispensable à la conduite du procédé selon la présente invention. En général, la quantité d'eau à mettre en oeuvre est telle que le rapport molaire eau/acide(s) penténique(s) soit compris entre 1 et 10 (inclus).

Une quantité inférieure présente l'inconvénient de limiter la conversion. Une quantité supérieure n'est pas souhaitable en raison de la perte d'activité catalytique observée.

Selon l'une des caractéristiques essentielles de la présente invention, la réaction est mise en oeuvre dans au moins un solvant choisi dans le groupe constitué par les hydrocarbures aliphatiques ou cycloaliphatiques, saturés, les hydrocarbures aromatiques et leurs dérivés chlorés.

La nature précise du solvant choisi dans le groupe précité n'est pas critique dans le cadre du présent procédé, pour autant que celui-ci soit à l'état liquide dans les conditions réactionnelles.

A titre d'exemple de tels solvants on peut citer : le benzène, le toluène, le chlorobenzène, le chlorure de méthylène, l'hexane et le cyclohexane.

Le toluène et le chlorobenzène conviennent plus particulièrement à la mise en oeuvre du procédé selon l'invention.

La quantité de solvant présente dans le milieu réactionnel peut varier dans de larges limites, par exemple de 10 à 99 % (inclus) en volume du milieu réactionnel. De préférence cette quantité est comprise entre 30 et 90 % en volume (inclus).

Selon une autre caractéristique essentielle du présent procédé, la pression partielle de l'oxyde de carbone, mesurée à 25°C, est inférieure à 12 bar.

Lorsque la pression partielle de l'oxyde de carbone, mesurée à 25°C, est supérieure à cette valeur la sélectivité en diacides linéaire parmi les diesters (taux de linéarité) chute considérablement.

Un minimum de pression partielle de l'oxyde de carbone de 0,5 bar (mesurée à 25°C) est préconisé.

De préférence, la pression partielle de l'oxyde de carbone, mesurée à 25°C, est inférieure ou égale à 8 bar.

On peut utiliser de l'oxyde de carbone sensiblement pur ou de qualité technique, tel qu'il se présente dans le commerce.

Comme indiqué en tête du présent mémoire, la température de réaction est comprise entre 100 et 240°C. Pour une bonne mise en oeuvre de la présente invention la température est comprise entre 160 et 190°C (inclus).

La réaction est conduite sous pression supérieure à la pression atmosphérique et, généralement, en phase liquide.

La pression totale peut varier dans certaines limites qui dépendront du mode opératoire retenu, de la pression partielle de l'oxyde de carbone et de celles des constituants du milieu réactionnel à la température réac-

EP 0 478 472 B1

tionnelle choisie et, le cas échéant de la pression autogène du (ou des) acide(s) penténique(s) présents.

Le milieu réactionnel renferme au moins un solvant choisi dans le groupe précité, de l'eau, une ou des sources de rhodium, un ou des promoteurs iodés et, le cas échéant tout ou partie du ou des acides penténiques engagés et des produits de la réaction.

En fin de réaction ou du temps imparti à celle-ci, on sépare l'acide adipique par tout moyen approprié par exemple par cristallisation.

Les exemples ci-après illustrent la présente invention.

Exemple 1 :

Dans un autoclave en acier inoxydable (HASTELLOY B2) de 125 cm3 préalablement purgé à l'argon, on introduit :

160 mg (0,65 mmol) de rhodium sous forme de [RhCl(COD)]$_2$
0,7 g (5 mmol) de $CH_3I$
2 g (110 mmol) d'eau
5 g ( 50 mmol) d'acide pentène-3 oïque
50 cm$^3$ de toluène

L'autoclave est fermé hermétiquement, placé dans un four agité et raccordé à l'alimentation de gaz sous pression. On admet à froid 2 bar de CO et on chauffe à 190°C en 30 minutes. Lorsque cette température est atteinte, on régule la pression à 20 bar.

Après une durée de réaction de 20 minutes, l'absorption de CO a cessé ; l'autoclave est alors refroidi et dégazé.

La solution réactionnelle est analysée par chromatographie en phase gazeuse et par chromatographie liquide haute performance.

Les quantités de produits formés (rendement molaire par rapport à l'acide pentène-3 oïque chargé) sont les suivantes :

acide valérique        (Pa) :  ⌇⌇⌇ COOH        =    3   %

acide pentène-2 oïque (P2) :  ⌇⌇⌇ COOH        =    8   %

méthyl-4 butyrolactone (M4L)  :  ⌇⌇⌇ = O        =    8   %

acide éthylsuccinique  (A3) :  HOOC ⌇⌇⌇ COOH        =    5  %

acide méthylglutarique (A2) :  HOOC ⌇⌇⌇ COOH        =   27  %

acide adipique         (A1) : HOOC ⌇⌇⌇ COOH        =   47  %

Le taux de linéarité (L) est de 60 %
Le taux de transformation de l'acide pentène-3 oïque (TT) est de 100 %.

Exemple 2 à 5 ; Essais Témoins (a) à (d) :

Dans l'autoclave et selon le mode opératoire, décrits pour l'exemple 1, on réalise une première série d'essais, à 175°C et en modifiant la pression partielle de l'oxyde de carbone et/ou la nature du solvant utilisé.

Les conditions particulières ainsi que les résultats obtenus toutes conditions égales par ailleurs sont ras-

4

semblés dans le tableau (I) ci-après, dans lequel les conventions utilisées sont les mêmes que pour l'exemple 1 et t représente la durée de réaction en température.

## TABLEAU I

| Réf. | Solvant | P(CO) bar | t mn | TT % | Al % | L % | Pa % |
|------|---------|-----------|------|------|------|-----|------|
| 2 | Toluène | 3 | 120 | 32 | 10 | 62 | < 1 |
| 3 | " | 11 | 20 | 100 | 45 | 53 | 3 |
| a | " | 18 | 20 | 100 | 36 | 43 | 3 |
| b | " | 71 | 20 | 100 | 19 | 24 | 2 |
| 4 | Chloro-benzène | 4 | 30 | 100 | 52 | 70 | 5 |
| c | " | 22 | 20 | 100 | 31 | 35 | 2 |
| 5 | Chlorure de Méthylène | 8 | 20 | 100 | 55 | 61 | 2 |
| d | " | 25 | 20 | 100 | 38 | 44 | 1 |

Ces résultats mettent en évidence le rôle déterminant d'une faible pression partielle de CO sur le taux de linéarité (L).

Exemple 6 :

Dans l'autoclave et selon le mode opératoire décrits ci-avant, on reproduit l'exemple 5 à ceci près que la charge contient 50 mmol d'acide pentène-4 oïque au lieu de l'acide pentène-3 oïque.
Après 30 mn de réaction toutes conditions égales par ailleurs, les résultats obtenus sont les suivants :
. TT = 100 %
. A1 = 69 %
. L = 85 %
. Pa = 1 %

Exemple 7 :

Dans l'autoclave et selon le mode opératoire décrits ci-avant, on reproduit l'exemple 5 à ceci près que la charge contient 50 mmol d'acide pentène-2 oïque au lieu de l'acide pentène-3 oïque.
Après 16 h de réaction toutes conditions égales par ailleurs, les résultats obtenus sont les suivants :
. TT = 62 %
. A1 = 36 %
. L = 60 %
. Pa = 26 %

Exemple 8 :

Dans l'autoclave et selon le mode opératoire, décrits ci-avant on reproduit l'exemple 4 en ne modifiant que la température (150°C).
Après 60 mn de réaction les résultats obtenus sont les suivants :
. TT = 100 %
. A1 = 42 %
. L = 46 %

**Revendications**

1. Procédé de préparation de l'acide adipique par réaction de l'eau et de l'oxyde de carbone sur au moins un acide penténique, en présence d'un catalyseur à base de rhodium et d'au moins un promoteur iodé, à une température comprise entre 100 et 240°C, sous une pression supérieure à la pression atmosphérique caractérisé en ce que

    a) la réaction est mise en oeuvre dans au moins un solvant choisi dans le groupe constitué par les hydrocarbures aliphatiques ou cycloaliphatiques, saturés, les hydrocarbures aromatiques et les hydrocarbures cycloaliphatiques ou aromatiques chlorés,
    b) la pression partielle de l'oxyde de carbone mesurée à 25°C est inférieure à 12 bar.

2. Procédé de préparation de l'acide adipique par réaction de l'eau et de l'oxyde de carbone sur au moins un acide penténique, en présence d'un catalyseur à base de rhodium et d'au moins un promoteur iodé, à une température comprise entre 100 et 240°C, sous une pression supérieure à la pression atmosphérique caractérisé en ce que

    a) la réaction est mise en oeuvre dans un hydrocarbure aliphatique chloré,
    b) la pression partielle de l'oxyde de carbone mesurée à 25°C est inférieure ou égale à 8 bar.

3. Procédé selon la revendication 1, caractérisé en ce que le solvant est choisi parmi le toluène et le chlorobenzène.

4. Procédé selon la revendication 2, caractérisé en ce que le solvant est le chlorure de méthylène.

5. Procédé selon la revendication 1 à 4, caractérisé en ce que le solvant représente au minimum 10 % en volume du milieu réactionnel liquide.

6. Procédé selon la revendication 5, caractérisé en ce que le solvant représente de 30 à 90 (inclus) % en volume du milieu réactionnel liquide.

7. Procédé selon l'une quelconque des revendications précédentes, caractérisé en ce que la concentration en rhodium dans le milieu réactionnel est comprise entre $10^{-3}$ et $10^{-1}$ mol/l (inclus).

8. Procédé selon l'une quelconque des revendications précédentes, caractérisé en ce que le rapport molaire I/Rh est supérieur ou égal à 0,1.

9. Procédé selon l'une quelconque des revendications précédentes caractérisé en ce que le rapport molaire I/Rh est inférieur ou égal à 20.

10. Procédé selon l'une quelconque des revendications précédentes, caractérisé en ce que le rapport molaire eau/acide(s) penténique(s) est compris entre 1 et 10 (inclus).

11. Procédé selon l'une quelconque des revendications précédentes, caractérisé en ce que la température de réaction est comprise entre 160 et 190°C.

12. Procédé selon l'une des revendications 1 et 3 à 11, caractérisé en ce que la pression partielle de l'oxyde de carbone, mesurée à 25°C, est inférieure ou égale à 8 bar.

**Patentansprüche**

1. Verfahren zur Herstellung von Ädipinsäure durch Reaktion von Wasser und Kohlenmonoxid mit wenigstens einer Pentensäure in Gegenwart eines auf Rhodium basierenden Katalysators und wenigstens eines iodierten Promotors bei einer Temperatur zwischen 100 und 240°C unter einem über dem Atmosphärendruck liegenden Druck, dadurch gekennzeichnet, daß

    a) die Reaktion in wenigstens einem Lösungsmittel durchgeführt wird. ausgewählt aus der aus den gesättigten aliphatischen oder cycloaliphatischen Kohlenwasserstoffen, den aromatischen Kohlenwasserstoffen und den chlorierten cycloaliphatischen oder aromatischen Kohlenwasserstoffen bestehenden Gruppe,
    b) der Partialdruck des Kohlenmonoxids, bei 25°C gemessen, kleiner als 12 bar ist.

**2.** Verfahren zur Herstellung von Adipinsäure durch Reaktion von Wasser und Kohlenmonoxid mit wenigstens einer Pentensäure in Gegenwart eines auf Rhodium basierenden Katalysators und wenigstens eines iodierten Promotors bei einer Temperatur zwischen 100 und 240°C unter einem über dem Atmosphärendruck liegenden Druck, dadurch gekennzeichnet. daß
a) die Reaktion in einem chlorierten aliphatischen Kohlenwasserstoff durchgeführt wird.
b) der Partialdruck des Kohlenmonoxids, bei 25°C gemessen, kleiner als oder gleich 8 bar ist.

**3.** Verfahren nach Anspruch 1, dadurch gekennzeichnet, daß das Lösungsmittel aus dem Toluol und dem Chlorbenzol ausgewählt ist.

**4.** Verfahren nach Anspruch 2, dardurch gekennzeichnet, daß das Lösungsmittel Methylenchlorid ist.

**5.** Verfahren nach Anspruch 1 bis 4, dadurch gekennzeichnet, daß das Lösungsmittel mindestens 10 Volumenprozent das flüssigen Reaktionsmediums ausmacht.

**6.** Verfahren nach Anspruch 5, dadurch gekennzeichnet, daß das Lösungsmittel zwischen 30 und 90 (einschließlich) Volumenprozent des flüssigen Reaktionsmediums ausmacht.

**7.** Verfahren nach einem beliebigen der vorhergehenden Ansprüche, dadurch gekennzeichnet, daß die Konzentration an Rhodium in dem Reaktionsmedium zwischen $10^{-3}$ und $10^{-1}$ mol/l (einschließlich) liegt.

**8.** Verfahren nach einem beliebigen der vorhergehenden Ansprüche, dadurch gekennzeichnet, daß das molare Verhältnis I/Rh größer als oder gleich 0.1 ist.

**9.** Verfahren nach einem beliebigen der vorhergehenden Ansprüche, dadurch gekennzeichnet, daß das molare Verhältnis I/Rh kleiner als oder gleich 20 ist.

**10.** Verfahren nach einem beliebigen der vorhergehenden Ansprüche, dadurch gekennzeichnet, daß das molare Verhältnis Wasser/Pentensäure(n) zwischen 1 und 10 (einschließlich) liegt.

**11.** Verfahren nach einem beliebigen der vorhergehenden Ansprüche, dadurch gekennzeichnet. daß die Reaktionstemperatur zwischen 160 und 190°C liegt.

**12.** Verfahren nach einem der Ansprüche 1 und 3 bis 11, dadurch gekennzeichnet. daß der Partialdruck des Kohlenmonoxids, bei 25°C gemessen, kleiner als oder gleich 8 bar ist.

## Claims

**1.** Process for the preparation of adipic acid by reaction of water and carbon monoxide with at least one pentenic acid in the presence of a rhodium-based catalyst and of at least one iodine-containing promoter, at a temperature of between 100 and 240°C, at a pressure higher than atmospheric pressure, characterized in that
a) the reaction is performed in at least one solvent chosen from the group consisting of saturated aliphatic or cycloaliphatic hydrocarbons, aromatic hydrocarbons and chlorinated cycloaliphatic or aromatic hydrocarbons,
b) the partial pressure of carbon monoxide, measured at 25°C, is lower than 12 bars.

**2.** Process for the preparation of adipic acid by reaction of water and carbon monoxide with at least one pentenic acid, in the presence of a rhodium-based catalyst and of at least one iodine-containing promoter, at a temperature of between 100 and 240°C, at a pressure higher than atmospheric pressure, characterized in that
a) the reaction is performed in a chlorinated aliphatic hydrocarbon,
b) the partial pressure of carbon monoxide, measured at 25°C, is lower than or equal to 8 bars.

**3.** Process according to Claim 1, characterized in that the solvent is chosen from toluene and chlorobenzene.

**4.** Process according to Claim 2, characterized in that the solvent is methylene chloride.

**5.** Process according to Claim 1 to 4, characterized in that the solvent represents at least 10 % by volume of the liquid reaction mixture.

6. Process according to Claim 5, characterized in that the solvent represents from 30 to 90 (inclusive) % by volume of the liquid reaction mixture.

7. Process according to any one of the preceding claims, characterized in that the rhodium concentration in the reaction mixture is between $10^{-3}$ and $10^{-1}$ mol/l (inclusive).

8. Process according to any one of the preceding claims, characterized in that the I/Rh molar ratio is higher than or equal to 0.1.

9. Process according to any one of the preceding claims, characterized in that the I/Rh molar ratio is lower than or equal to 20.

10. Process according to any one of the preceding claims, characterized in that the molar ratio water/pentenic acid(s) is between 1 and 10 (inclusive).

11. Process according to any one of the preceding claims, characterized in that the reaction temperature is between 160 and 190°C.

12. Process according to one of Claims 1 and 3 to 11, characterized in that the partial pressure of carbon monoxide, measured at 25°C, is lower than or equal to 8 bars.